(19) Europäisches Patentamt
European Patent Office
Office européen des brevets

(11) Publication number: **0 522 599 B1**

(12) **EUROPEAN PATENT SPECIFICATION**

(45) Date of publication of patent specification: **28.12.94**

(51) Int. Cl.[5]: **C07D 471/04**, A61K 31/435

(21) Application number: **92115805.1**

(22) Date of filing: **21.05.86**

(60) Publication number of the earlier application in accordance with Art.76 EPC: **0 202 661**

(54) **New method for the isolation of castanospermine.**

(30) Priority: **24.05.85 US 738127**

(43) Date of publication of application: **13.01.93 Bulletin 93/02**

(45) Publication of the grant of the patent: **28.12.94 Bulletin 94/52**

(84) Designated Contracting States: **AT BE CH DE FR GB IT LI LU NL SE**

(56) References cited:

**PHYTOCHEMISTRY, vol. 20, no. 4, 1981, pages 811-814; L.D. HOHENSCHUTZ et al.: "Castanospermine, A 1,6,7,8-tetrahydroxyoctahydroindolizine alkaloid, from seeds of Castanospermum australe"**

(73) Proprietor: **MERRELL DOW PHARMACEUTICALS INC.**
**2110 East Galbraith Road**
**Cincinnati**
**Ohio 45215 (US)**

(72) Inventor: **Liu, Paul S.**
**7320 Lakeside Drive**
**Indianapolis**
**Indiana 46278 (US)**
Inventor: **Rhinehart, Barry L.**
**9021 Jackson**
**Indianapolis**
**Indiana 46231 (US)**

(74) Representative: **Macchetta, Francesco et al**
**GRUPPO LEPETIT S.p.A.**
**Patent and Trademark Department**
**Via Roberto Lepetit, 34**
**I-21040 Gerenzano (Varese) (IT)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Castanospermine is an alkaloid having the following formula:

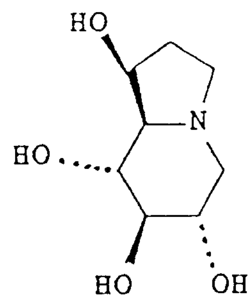

Systematically, this compound is named (1S,6S,7R,8R,8aR)-1,6,7,8-tetrahydroxyindolizidine.

The isolation of this compound from Castanospermum australe has been described by L.D. Hohenshutz, et al., Phytochemistry, 20, 811 (1981). The procedure involves a tedious extraction and a subsequent washing with pyridine and only small quantities of the desired product are obtained. R.C. Bernotas, et al., Tetrahedron Letters, 25, 165 (1984) obtained castanospermine by total synthesis and thereby established the absolute configuration of the compound.

Several publications have appeared which describe castanospermine as an inhibitor of a number of plant enzymes. See Y.T. Pan, et al., Biochemistry, 22, 3975 (1983); R. Saul, et al., Archives of Biochemistry and Biophysics, 221, 593 (1983); R. Saul, et al., Archives of Biochemistry and Biophysics, 230, 668 (1984). More recently, Saul et al., Proc. Nat'l. Acad. Sci. USA, 82, 93 (1985) have indicated that castanospermine inhibits intestinal glycosidases (i.e., maltase and sucrase) in rats when administered by injection and that diarrhea and high levels of intestinal bacterial flora were observed when high doses were administered. However, they gave no indication that castanospermine would be useful for any purposes as a result of these facts.

It has also been found that castanospermine possesses digestive enzyme inhibitory properties and thus can be used as an antidiabetic agent and as an inhibitor of increased lipid biosynthesis. Thus, when carbohydrate is ingested either as glucose or in a form such as maltose, sucrose or starch in food or drink, the blood glucose level rises to elevated concentrations. In healthy subjects, this hyperglycemic state quickly returns to normal, the glucose in the blood being rapidly metabolized and stored and/or utilized by the organism. In diabetes mellitus, however, the glucose tolerance of the patient is lowered and the abnormally high blood sugar levels which develop remain elevated for prolonged periods of time. A similar response to that seen in man can also be observed in other animals, including livestock, poultry, pet animals and laboratory animals. Such a condition can be described as postprandial hyperglycemia. One method for treating such a condition would be by administration of some substance which would prevent the conversion of complex sugars to glucose and thus prevent the development of the excessive glucose levels. In the present invention, it has been found that, where the high levels of glucose are a result of the hydrolysis of complex sugars, administration of castanospermine inhibits the initial formation of glucose in the blood and thus makes it possible to avoid the problems which would be associated with prolonged high levels of blood sugar.

Equivalent to castanospermine for these purposes are the salts of castanospermine with pharmaceutically acceptable acids and, particularly, with inorganic acids such as hydrochloric acid, hydrobromic acid, sulfuric acid and phosphoric acid; and with organic acids such as acetic acid, propionic acid, methanesulfonic acid and p-toluenesulfonic acid.

The present invention is directed to a new process for the isolation of castanospermine from Castanospermum australe by a procedure that avoids the tedious extraction procedure and the use of the obnoxious solvent pyridine as described in the published procedure mentioned earlier while providing the desired product in greatly improved overall yields. Details of this procedure are set forth in the example.

The following example is presented to illustrate the present invention.

EXAMPLE 1

Commercially available mature seeds of Castanospermum australe (300 g, wet weight) were ground and continuously extracted for 24 hours in a Soxhlet apparatus with 1.0 l of a 3:7 (v/v) mixture of water and 2-propanol. The extract was concentrated in vacuo to a final volume of 250 ml and was filtered. After extraction with petroleum ether (10 x 100 ml), the aqueous extract was subjected to column chromatography with Dowex 50W-X4 ($H^+$) ion-exchange resin (2.5 cm x 32 cm). The column was washed with distilled water (200 ml) and the desired alkaloid was eluted with 400 ml of 2 N aqueous ammonium hydroxide. The basic eluate was concentrated in vacuo to 125 ml and applied to another column containing Dowex 1-X4-($OH^-$) ion-exchange resin (2.5 cm x 20 cm). The column was eluted with 125 ml of water and the eluate was concentrated to a final volume of 30 ml. Trituration of the concentrate with 300 ml of acetone yielded 3.5 g of castanospermine as colorless crystals (1.2%, calculation on the basis of the weight of fresh seeds). The isolated crystals had the following physical properties:

mp: 212-225 °C

| Elemental analysis: | | | |
|---|---|---|---|
| Found:<br>Calc for $C_8H_{15}NO_4$: | C, 50.53%;<br>C, 50.79%; | H, 8.04%;<br>H, 7.94%; | N, 7.26%.<br>N, 7.41%. |

Optical rotation = $[\alpha]_D^{25}$ + 75.5° (c 1,72, $H_2O$)
Mass Spectral Data: 190 ($MH^+$), 172 ($MH^+$-$H_2O$).

**Claims**

1. A process for obtaining castanospermine, i.e., 1S,6S,7R,8R,8aR-1,6,7,8-tetrahydroxyindolizidine of formula

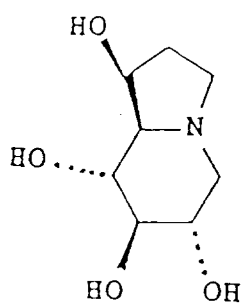

or a salt thereof with a pharmaceutically acceptable acid from the seeds of *Castanospermum australe* which comprises:
(a) extracting the ground seeds with a mixture of water and 2-propanol;
(b) washing the extract with petroleum ether;
(c) chromatographing the solution on an acid resin with elution with ammonium hydroxide;
(d) rechromatographing the eluate on a basic resin with elution with water; and
(e) concentration of the aqueous eluate followed by trituration with acetone to give castanospermine.

2. A process according to claim 1, wherein the extraction is conducted with a 3:7 (v/v) mixture of water and 2-propanol.

**Patentansprüche**

1. Verfahren zur Isolierung von Castanospermin, d.h. von 1S,6S,7R,8R,8aR-1,6,7,8-tetrahydroxyindolizidin der Formel

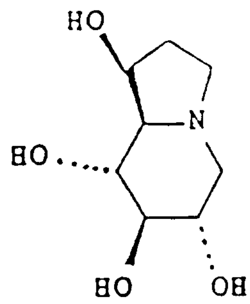

oder eines Salzes davon mit einer pharmazeutisch verträglichen Säure aus den Samen von *Castanospermum australe*, das umfaßt:

(a) Extrahieren der gemahlenen Samen mit einem Gemisch aus Wasser und 2-Propanol;
(b) Waschen des Extrakts mit Petrolether;
(c) Chromatographieren der Lösung auf einem sauren Harz unter Eluieren mit Ammoniumhydroxid;
(d) Erneutes Chromatographieren des Eluats auf einem basischen Harz unter Eluieren mit Wasser; und
(e) Einengen des wäßrigen Eluats und anschließendes Verreiben mit Aceton, wobei sich Castanospermin ergibt.

**2.** Verfahren nach Anspruch 1, wobei die Extraktion mit einem 3:7 (v/v) Gemisch aus Wasser und 2-Propanol durchgeführt wird.

**Revendications**

**1.** Procédé de préparation de castanospermine, soit la 1S,6S,7R,8R,8aR-1,6,7,8-tétrahydroxyindolizidine de formule:

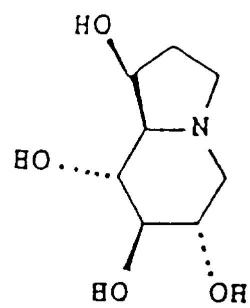

ou un sel de celle-ci avec un acide pharmaceutiquement acceptable à partir de graines de *Castanospermum australe* , lequel comprend:

(a) l'extraction de graines moulues par un mélange d'eau et de 2-propanol;
(b) le lavage de l'extrait par l'éther de pétrole;
(c) la chromatographie de la solution obtenue sur une résine acide en éluant par de l'hydroxyde d'ammonium;
(d) une chromatographie supplémentaire de l'éluat sur une résine basique en éluant par de l'eau; et
(e) la concentration de l'éluat aqueux suivie d'une trituration dans l'acétone pour donner la castanospermine.

**2.** Procédé selon la revendication 1, dans lequel l'extraction est réalisée par un mélange 3:7 (v/v) d'eau et de 2-propanol.